# EUROPEAN PATENT APPLICATION

(11) **EP 1 810 686 A1**
(43) Date of publication of application: **25.07.2007**
(21) Application number: 06075119.5
(22) Date of filing: 19.01.2006
(51) Int. Cl.: A61K 38/55, C07K 16/40, G01N 33/53

(54) **Means and methods for modulating stem cell mobilization**

(71) Applicant: Academisch Ziekenhuis Leiden, 2333 ZA Leiden (NL)
(72) Inventor: Fibbe, Willem Eduard, 2161 EP Lisse (NL); van Pel, Melissa, 2133 CS Hoofddorp (NL)
(74) Representative: Winckels, Johannes Hubertus F.

(57) **Abstract**

Mobilization of stem cells in individuals is currently used in methods for their collection and in methods for therapeutically intervening in disease processes in the human body. The present invention provides means and methods for increasing numbers of mobilized stem cells and provides uses therefore.

## Description

The invention relates to stem cell technology, more in particular to mobilization of stem cells in individuals.

Since a number of years, cytokine-mobilized blood stem cells have become the primary source of hematopoietic stem- and progenitor cells (HSC/HPC) for transplantation in humans (1-3). In mice, many cytokines and chemokines induce HSC/HPC mobilization after prolonged administration, such as granulocyte-colony stimulating factor (G-CSF), whereas only few induce HSC/HPC mobilization upon a single injection, such as IL-8 (4-6). Despite the wide applications of mobilized HSC/HPC, the mechanisms that contribute to the release of HSC/HPC into the peripheral blood are not completely understood. However, evidence has accumulated that HSC/HPC mobilization is a multistep process in which multiple cell types and molecules are involved.

Under steady-state conditions, HSC/HPC are retained in the bone marrow (BM) micro-environment via cytoadhesive molecules. Adhesion blocking experiments have revealed that the Very Late Antigen (VLA)-4/Vascular Cell Adhesion Molecule (VCAM)-1, VLA-5/fibronectin, and b2 integrins/intercellular adhesion molecule-1 (ICAM-1) pathways play a role in the attachment of CD34+ cells to stromal cells (7, 8). Interruption of c-Kit/SCF, CXCR-4/stromal-derived factor-1 (SDF-1, CXCL12) and VLA-4/VCAM-1 has been shown to be involved in HSC/HPC mobilization (9-15).

Previous studies from our laboratory have demonstrated that a single injection of the CXC chemokine Interleukin (IL)-8, which is a chemoattractant and activator of neutrophils, induces rapid (15-30 minutes) mobilization of HPC and repopulating HSC (6, 16). Neutrophils have been shown to be indispensable for this process, since IL-8-induced HSC/HPC mobilization is completely abolished in mice rendered neutropenic following administration of a depleting anti-GR-1 antibody (17). In addition, neutralizing antibodies against the b2 integrins Lymphocyte Function Associated molecule (LFA)-1 and Mac-1 (CD11b) completely prevented IL-8-induced HSC/HPC mobilization (18). This effect was not due to direct targeting of hematopoietic progenitor cells and stem cells, since b2 integrins are not expressed on the surface of hematopoietic progenitor cells and stem cells (19, 20). Beta2 integrins are critically involved in firm attachment of neutrophils to the endothelium, which forms a crucial step in enabling degranulation following activation by IL-8. These observations indicate that b2 integrin antibodies abolished IL-8-induced HSC/HPC mobilization by preventing adhesion, attachment and degranulation of neutrophils. The role of neutrophils was further demonstrated by the observation that HSC/HPC mobilization in response to IL-8 in neutropenic hosts was restored upon the infusion of purified neutrophils (17). In addition, proteases that are present in the granules of neutrophils have been shown to be active in G-CSF -and IL-8-induced HSC/HPC mobilization (12, 13, 21).

In the present invention it was observed that low dose total body irradiation (TBI) inhibits IL-8-induced HSC/HPC mobilization. We show that bone marrow extracellular extracts (BMEE) from low dose TBI inhibit elastase, an enzyme that is released by activated neutrophils. We show that this phenomenon is associated with the induction of the protease inhibitor serpina 1 (also known as α-1 antitrypsin or α-1 proteinase inhibitor) in the BM. We show that serpina1 is a potent inhibitor of HSC/HPC mobilization following exogenous administration and we demonstrate that radiation-induced serpina1 is responsible for the inhibition of mobilization observed after low-dose TBI. We further demonstrate that stem cell mobilization is enhanced when individuals are provided with a binding body that is specific for serpina1. Thus in one aspect the invention provides a method for modulating mobility of a stem cell in an individual, said method comprising modulating elastase activity in the vicinity of said stem cell. By providing elastase activity stem cell mobility is enhanced, whereas inhibiting elastase activity results in decreased mobility. Mobility of a stem cell in an individual is preferably measured by determining their presence in circulating blood. An elevated number of stem cells in the circulating blood indicates increased mobility, whereas a decreased number of stem cells therein indicates decreased mobility. Stem cells are preferably measured by their functional properties, i.e. being undifferentiated, pluripotent and having extensive self-renewal capabilities. Stem cells can also be detected in other ways for instance through the presence of specific markers or combinations of markers on the cell surface. As stem cells are sometimes not very numerous in cell populations, derivative assays have been developed to estimate their presence and their quantity in a population of cells. Such derivative assays can of course also be used in the present invention. Non-limiting examples of such derivative assays are assays that detect progeny of stem cells in various stages of differentiation. Typically colony forming cells, cobblestone forming cells or long term culture initiating cells are used as derivative assays. These are typically used when the source of cells that is tested for the presence of stem cells is a familiar source wherein such derivative assays are routinely used to estimate the quantity of stem cells. Blood from a normal individual or from an individual that is treated for mobilization is such a familiar source.

In the present invention it was found that stem cell mobility in an individual is regulated by, among others, a balance between elastase and an inhibitor therefore. For example, mobility of stem cells is decreased when inhibitors for elastase are upregulated. Said mobility is preferably increased by increasing elastase activity in the vicinity of said stem cell. The latter can be achieved in various ways. In a preferred embodiment this is achieved by providing said individual with an inhibitor of a protease inhibitor. The protease activity of elastase can be inhibited by a number of different protease inhibitors. Irrespective of whether the effect is related to elastase or not, it has been observed that inhibiting such inhibitors in an individual results in increased mobility of stem cells in said individual. The invention therefore in another aspect provides a method for increasing mobility of a stem cell in an individual, comprising providing said individual with an inhibitor of a protease inhibitor.

Several kinds of inhibitors of a protease inhibitor can be generated. In a preferred embodiment said inhibitor is a binding body that is specific for said protease inhibitor. Nowadays many different binding bodies exist. The binding body may be large such as an antibody, or may be small such as a small molecule. Non-limiting examples of such small molecules are single chain Fv fragments, monobodies, other single variable domain molecules such as VHH and VH domains, and the like. A binding body of the invention is preferably a proteinaceous binding body. Antibodies and their functional parts, derivatives and analogues are preferred binding bodies of the invention. A functional part, derivative and/or analogue of an antibody comprises at least the same binding specificity in kind, not necessarily in amount as an antibody of the invention. The binding specificity of an antibody is located in the variable regions. These regions have a highly variable part (the CDR regions) and a relatively constant part (the framework region). The binding specificity of an antibody is predominantly correlated with the specific amino acid sequence of the CDR regions, and particularly that of the CDR3 region. CDR3 and/or the other CDRs of an antibody can be grafted onto different amino acid sequences. When the receiving amino acid sequence provides the same structural context as the framework region in the original antibody, the newly synthesized molecule comprises the same binding specificity in kind as the antibody that donated the one or more CDRs. This newly synthesized molecule comprises at least a functional part of the antibody that donated said one or more CDR. Many other parts can be generated from an antibody. Non-limiting examples of such parts are FAB-fragments (1 or 2), single chain variable fragments (scFv) and so-called monobodies or VHH. All these parts share a common theme in that they comprise at least the CDR3 region of the donating antibody. CDRs can be grafted onto other antibodies or onto a part of an antibody. CDRs can also be grafted onto structures that resemble the three dimensional structure of an antibody (or part thereof) but that do not share extensive sequence homology with them. These latter structures can be obtained, for instance, by three dimensional modelling of antibodies. Amino acid substitutions can be tested for maintenance of the overall three dimensional structure of the molecule. The modelling is used to ensure that the three dimensional structure of at least the framework region is essentially unchanged. Such derivatives are also binding bodies of the invention. The general structure that provides the structural context for the CDRs (the framework region) is a structure that is used by many different proteins. Particularly by proteins that interact with other proteins. This general structure is also referred to as the Ig-fold. It has been shown that CDRs that are grafted onto such Ig-fold containing proteins provide the receiving protein with the same binding specificity in kind though not necessarily in amount, as the donating antibody. Such Ig-fold containing molecules comprising at least a CDR3 of an antibody of the invention are therefore functionally analogous to an antibody of the invention. Current methods allow the specific selection of a derivative of an antibody without first having to generate an antibody. Such derivatives having a specific binding peptide at the position that in a three dimensional model is in the same place as the CDR3 region of an antibody. In the present invention, these specific binding peptides are considered CDR3 regions.

Without being bound by theory it is thought that the binding of a binding body specific for serpina1 prevents interaction of a protease inhibitor with a protease in the vicinity of a stem cell, thereby allowing said protease to be more active. The binding body can prevent said interaction by simply blocking (part of) the protease interaction site on the protease inhibitor and/or it can alter the biodistribution of the protease inhibitor. In a preferred embodiment of the invention a protease inhibitor for which a specific binding body is provided belongs to the group identified by serpina1 and/or α2-MG. A binding body of the invention is preferably specific for serpina1. Providing a binding body specific to serpina1 mobilizes stem cells particularly efficient. In a preferred embodiment said stem cell is a bone marrow derived stem cell. Preferably a hemopoietic stem cell. Thus the invention further provides use of a binding body specific for a protease inhibitor, or a functional part, derivative and/or analogue of said antibody for stimulating mobility of a stem cell in an individual. An individual is preferably a mammal, more preferably a primate, preferably a human.

In a preferred embodiment said inhibitor of a protease inhibitor is provided to an individual which is being prepared for collection of mobilized stem cells. In the art many methods are known to mobilize stem cells. In a preferred embodiment said mobilization is performed by administering to said individual a growth factor/cytokine. Thus in a preferred embodiment said inhibitor is provided to said individual as part of a stem cell mobilization regime. In a preferred embodiment said inhibitor is administered to said individual at least one day prior to harvesting said stem cells. In a preferred embodiment said inhibitor is provided to said individual on the same day as that said growth factor/cytokine treatment is initiated. The invention thus further provides a kit comprising said one or more growth factors/cytokines and said inhibitor. Preferably said kit comprises at least C-GSF and said inhibitor. Further provided is a composition comprising a growth factor or cytokine for stimulating mobilization of stem cells in an individual and an inhibitor of serpina1. Preferably said growth factor comprises C-GSF.

In another aspect the invention provides a method for reducing the response time for growth factor/cytokine mediated mobilization of stem cells in an individual comprising providing said individual with said stem cells in an individual by means the

CD97 is a member of the EGF-TM7 family of class II seven-span transmembrane receptors and is broadly expressed on hematopoietic cells including lymphocytes, granulocytes, and monocytes. In the present invention it is demonstrated that CD97 is expressed on bone marrow derived stem cells and that CD97 can be used to distinguish collections of cells in a population of bone marrow derived cells that are enriched for said stem cells. Thus the present invention provides a method for identifying a cell in a population of cells, said method comprising contacting said population of cells with a binding body specific for CD97 and identifying said cell on the basis of the relative amount of binding body associated with said cell. Preferably said identifying comprises classifying cells in said population of cells as having a high amount, an intermediate amount or no detectable CD97 specific binding body associated therewith. The absence, presence or relative amount of detected CD97 can be used as a criterion for separating cells. Thus in a preferred embodiment said method further comprises separating said identified cell from at least part of the cells of said population of cells. Preferably said cell is identified through its classification as a cell having an intermediate level of CD97 specific binding body associated therewith. Stem cells contain an intermediate amount of CD97. Thus in a preferred embodiment said separated cell is a stem cell, preferably a bone marrow derived stem cell. In a preferred embodiment of the invention said method is used to separate hemopoietic stem cells from other bone marrow derived cells. Thus in a preferred embodiment said method further comprises contacting said population of cells a binding body specific for CD34 or homologue thereof, specific for CD38 or homologue thereof and/or specific for c-kit or homologue thereof. Preferably said method further comprises separating cells from said population on the basis of the relative amount of binding body specific for CD34, CD38 and/or c-kit or homologues thereof on said cells. In another preferred embodiment, a method of the invention is used for marking cells in said population of cells, to discriminate different populations therein, or to detect differences in the level of expression of CD97 in time. Thus a method of the invention preferably comprises use of a binding body specific for CD97 for marking a subset of cells in a population of cells. In yet another aspect, the invention provides a kit for separating stem cells from other cells in a population of cells comprising a binding body specific for CD97 or a homologue thereof. Separating cells from other cells can be done in various ways. A particularly convenient method for separating large numbers of cells incorporates a step wherein part of the cells in said population are associated with a solid surface. Said solid surface is typically associated with a binding body that is specific for a subset of cells in said population. The desired cell is either retained on the solid surface, or not. Thus said kit preferably further comprises a solid surface arranged for separating cells. Solid surfaces can be provided in the form of columns, (magnetic) beads or other surfaces. In a particularly preferred embodiment said binding body is associated with a solid surface. Thus in a preferred embodiment said binding body in said kit is associated with said solid surface. Said kit preferably further comprises a binding body for distinguishing stem cells from other cells in said population of cells.

### Examples

### Example 1

### Results

### Hematopoietic stem cell mobilization is inhibited at 24 hours following 0.5 Gy TBI

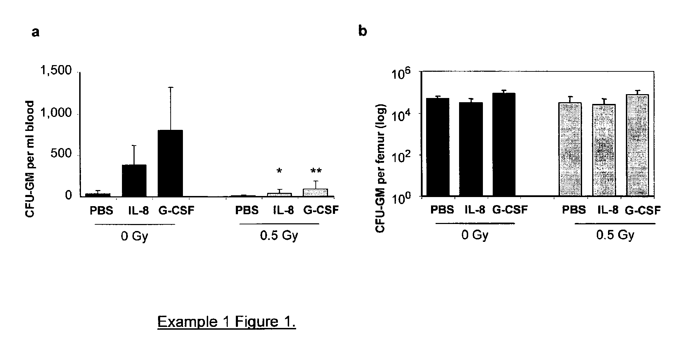

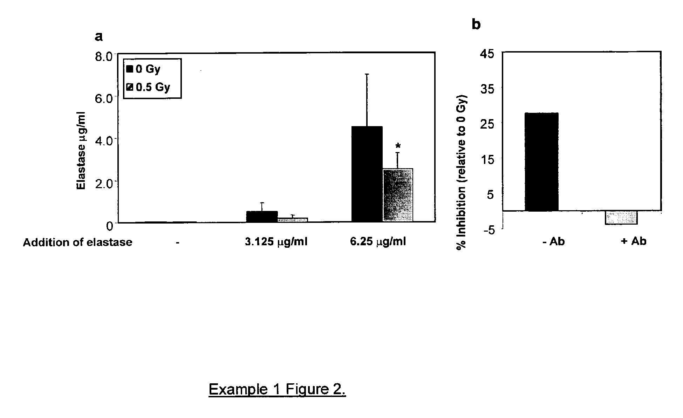

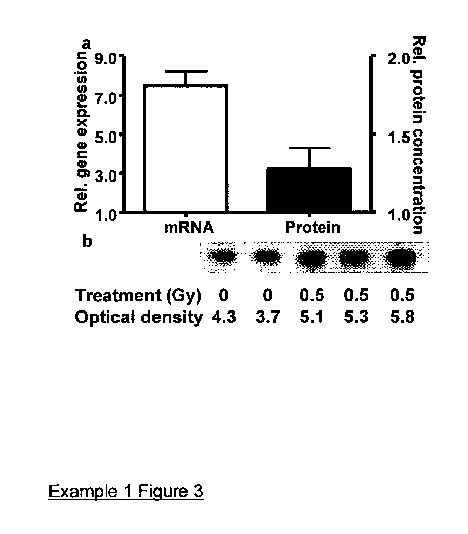

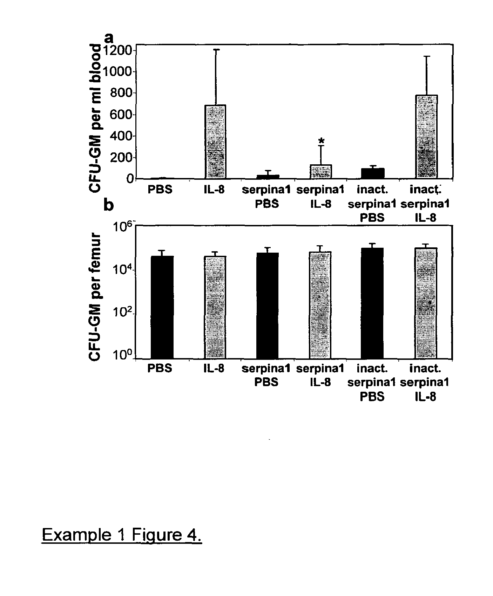

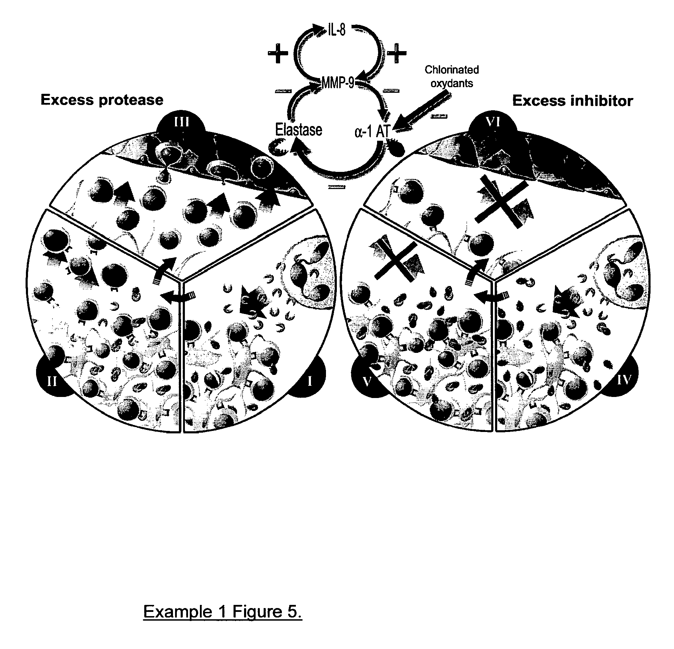

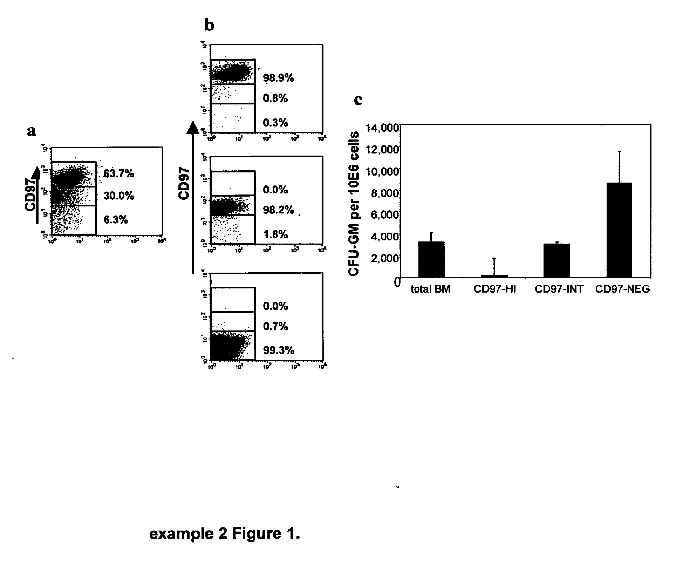

To study the effect of low dose irradiation on HSC/HPC mobilization, cohorts of mice received 0.5 Gy TBI and were injected with IL-8 at 24 hours following TBI. IL-8 -induced HSC/HPC mobilization to the peripheral blood was significantly impaired in irradiated mice compared to sham irradiated controls (388.1±233.3 vs. 37.1±51.5 CFU-GM for IL-8 vs. 0.5 Gy/IL-8 respectively, p<0.0001; figure 1a), whereas the number of progenitor cells in the BM was similar in all groups (p>0.5; figure 1b). HSC/HPC mobilization after 0.5 Gy TBI induced by three daily injections of G-CSF, initiated at 24 hours post TBI, was also significantly inhibited in irradiated (0.5 Gy) mice, compared to sham irradiated controls (800±513.6 vs. 91.9±96.7 CFU-GM for G-CSF vs. 0.5 Gy/G-CSF respectively, p<0.0001; figure 1a). No effect of low dose TBI on neutrophil numbers in peripheral blood and BM was observed (data not shown).

### BM extracellular extracts inhibit elastase activity

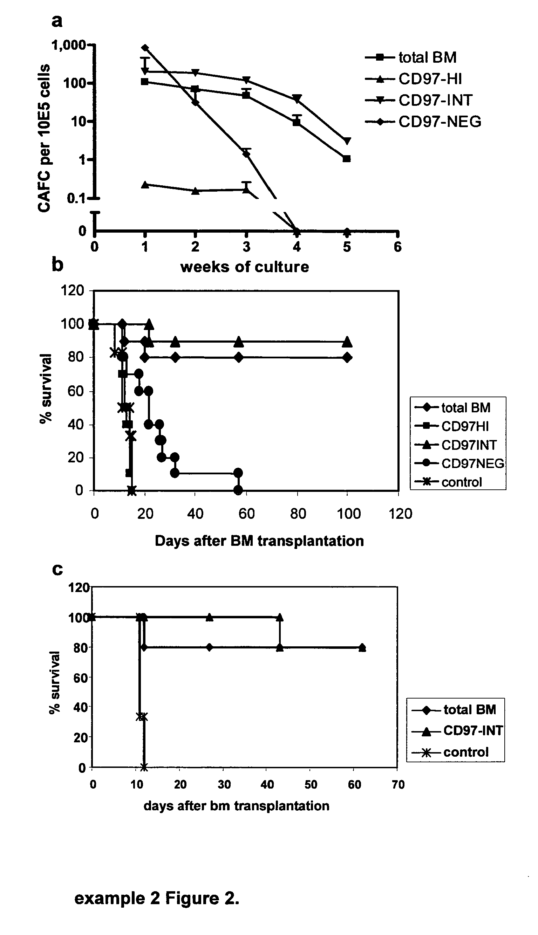

To determine the mechanism by which cytokine-induced HSC/HPC mobilization is inhibited after 0.5 Gy TBI, cohorts of mice received low dose (0.5 Gy) TBI or were sham-irradiated. Twenty-four hours following TBI, the mice were sacrificed and BM extracellular extracts (BMEE) were harvested. Subsequently, elastase activity was evaluated in BMEE using a chromogenic substrate conversion assay, specifically designed to detect elastase activity. No elastase activity was found in BMEE from irradiated mice (figure 2a). After addition of 3.125 or 6.25 µg/ml elastase to the BMEE obtained from low-dose (0.5 Gy) irradiated mice, elastase activity was significantly (p<0.05) reduced (60.3% and 44.1% respectively) compared to BMEE obtained from sham-irradiated control mice (figure 2a), indicating the presence of an elastase inhibitor in BMEE following low dose TBI.

### Inhibition of elastase activity can be reversed by serpina1 neutralizing antibodies

To assess whether the inhibition of elastase activity in BMEE obtained from low dose irradiated mice can be explained by the presence of serpina1 in the BMEE, neutralizing anti- serpina1 antibodies were incubated with BMEE obtained from low dose (0.5 Gy) irradiated mice (n=2) and activity of exogenous elastase was evaluated. As expected, after addition of 3.125 µg/ml elastase to the BMEE of 0.5 Gy irradiated mice, elastase activity was inhibited compared to BMEE obtained from unirradiated mice. However, following addition of anti- serpina1 antibodies, elastase activity was restored (figure 2b), indicating that serpina1 was responsible for the inhibitory activity in the BMEE obtained from low-dose irradiated mice.

### Induction of serpina1 mRNA and protein by low dose TBI

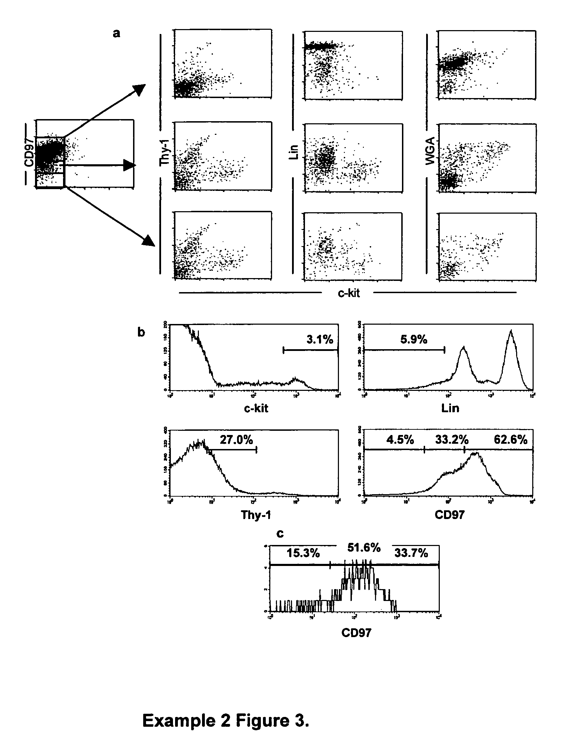

Since antibodies against serpina1 completely abolished the inhibitory effect of BMEE from irradiated mice, serpina1 was considered as the primary candidate serine protease inhibitor to inhibit elastase activity in our in-vitro system. To investigate whether serpina1 mRNA is induced following low dose (0.5 Gy) TBI, mRNA was isolated from 0.5 Gy-irradiated mice (n=2) at 24 hours following TBI. Relative to HPRT expression, serpina1 mRNA was increased 7-fold at 24 hours after 0.5 Gy TBI (figure 3a) and 50-fold after 3 Gy (data not shown). Relative to the more abundantly expressed housekeeping genes b-actin and GAPDH, a 20-fold and 7-fold increase of serpina1 mRNA was found respectively following 0.5 Gy TBI. These results indicate that serpina1 mRNA concentrations are increased following low dose TBI. To assess whether the upregulation of serpina1 mRNA also resulted in an increase in serpina1 protein concentration, the presence of serpina1 protein in BMEE was investigated by Western blotting. Ten µg of protein derived from BMEE , harvested from 0.5 Gy or sham-irradiated mice were loaded on an acryl/bisacrylamide gel and serpina1 was visualized by chemoluminescence (figure 3a). Next, the bands of each lane were quantitated and related to sham-irradiated controls on the same blot. The relative amount of serpina1 protein was significantly increased by 28% in BMEE from low dose irradiated (0.5 Gy) mice compared to sham irradiated controls (figure 3b; p<0.0001). In addition, upon 8.0 Gy TBI, the relative concentration of serpina1 protein was increased by 63% compared to sham-irradiated controls (data not shown). These results indicate that serpina1 protein is induced upon low dose TBI.

### IL-8-induced mobilization is inhibited by serpina1

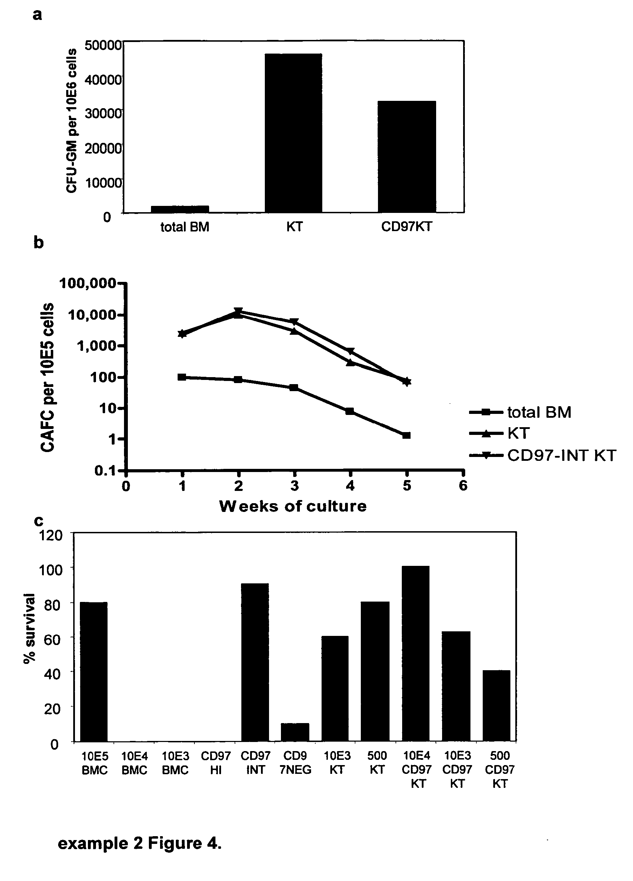

To further study a possible in-vivo role of serpina1 in IL-8-induced HSC/HPC mobilization, mice (n=14) were treated with serpina1 (300 µg/mouse) at 2 hours and at 5 minutes prior to IL-8 administration and the frequency of colony forming units (CFU-GM) in peripheral blood was evaluated. Administration of serpina1 prior to IL-8 injection completely prevented IL-8-induced HSC/HPC mobilization (PBS/IL-8, 685.1±522.1; n=7, serpina1 /PBS, 31.5±51.9; n=9 and serpina1 /IL-8, 131.1±180.9; n=14 CFU-GM per ml blood, p<0.05; figure 4a), whereas heat-inactivated serpina1 did not inhibit HSC/HPC mobilization (779.3±363.0; n=4). In addition, serpina1 alone did not induce HSC/HPC mobilization nor did it affect GM-CSF-induced colony formation in-vitro (data not shown). Furthermore, the number of colony forming cells in the BM remained unaltered; indicating that serpina1 administration did not affect colony forming cells in-vivo (figure 4b).

### Discussion

In the current report, we show that low dose (0.5 Gy) TBI inhibits IL-8-and G-CSF-induced HSC/HPC mobilization. The mechanism that underlies the inhibition of HSC/HPC mobilization was reversible since HSC/HPC mobilization was restored at 5 days following low dose TBI (data not shown). In addition, neither the progenitor cell content of the BM compartment nor the number of peripheral blood lymphocytes was affected by this low dose of irradiation. Since the release of proteases, including neutrophil elastase and Cathepsin G represents a final pathway in the induction of cytokine-induced HSC/HPC mobilization (10, 12, 13), we investigated a possible role of serine proteases or serine protease-inhibitors in this process. The inhibition of HSC/HPC mobilization was partially reversed upon transfusion of neutrophils obtained from unirradiated donor mice, which may indicate that neutrophils or neutrophil-derived proteases are involved in this process (data not shown). Furthermore, BMEE obtained from low-dose (0.5 Gy) irradiated mice were found to inhibit the bioactivity of elastase in a substrate conversion assay. Moreover, addition of anti- serpina1 antibodies to BMEE reversed the inhibition, showing that serpina1 was responsible for the inhibitory activity in the BMEE obtained from low-dose irradiated mice.
Serine proteases such as elastase are irreversibly inhibited by the serine protease inhibitor (serpin) serpina1 and a2-MG (28-30). Serpins belong to a single superfamily with highly conserved secondary structural elements and inhibit their target protease by covalent binding. This inhibition is irreversible and is required for effective control of the proteolytic cascade that may be initiated by the release of just a few protease molecules. In mammals, serpins play roles in a range of proteolytic processes including blood clotting, inflammation, and turnover of extracellular matrix (31). Alpha2-MG lures a protease with a residue domain that it is allowed to cleave. This activates the α2-MG molecule, leading to a conformational change by which it envelops the protease and prevents it attacking other substrates. However, molecules that consists of few amino acids may still enter the envelope and are subsequently degraded by the protease (30). Serpina1 functions by covalent binding and subsequent functional inhibition of target proteases. The interaction between serpina1 and its target is directed by the so-called reactive center loop (RCL), an approximately 20-residue domain that extends out from the body of the serpina1 polypeptide and determines the inhibitor's specificity. Serpina1, like a mousetrap, exists in a metastable state, and its energy is released by cleavage of the RCL. Elastase binds to and cleaves the RCL of serpina1, which then transposes the protease to the other end of the molecule. By this process, the protease is crushed against the serpin, resulting in a loss of structural integrity that ensures its destruction (32).
Since elastase that is inhibited by α2-MG still has the capacity to cleave the substrate that we use in our substrate inhibition assay, we could not measure the inhibition of elastase by α2-MG (30). Therefore, serpina1 was considered as the primary candidate serine protease inhibitor to inhibit elastase activity in our in-vitro system. Quantitative RT PCR of total BM cells confirmed that serpina1 mRNA was increased relative to housekeeping genes following low dose TBI. In addition, Western blot analysis indicated that the relative serpina1 protein concentrations were increased in BMEE derived from low dose irradiated mice. However, due to the lack of sufficient antibody for in-vivo use, we were unable to study whether radiation-induced inhibition of HSC/HPC mobilization can be reversed by treatment with anti- serpina1 antibodies in-vivo. Finally, the importance of serpina1 in HSC/HPC mobilization was shown by the absence of IL-8-induced HSC/HPC mobilization upon administration of serpina1 in-vivo. Heat-inactivated serpina1 had no effect on IL-8-induced HSC/HPC mobilization, pointing towards a specific role of active serpina1 in the inhibition of HSC/HPC mobilization rather than an artefact, due to non-specific effects.
Proteases have been shown to be regulatory mediators of cytokine-induced HSC/HPC mobilization. Studies from our laboratory in rhesus monkeys indicated a critical role for the matrix metalloproteinase-9 (MMP-9; gelatinase B) that is stored in the azurophilic granules of neutrophils. IL-8-induced HSC/HPC mobilization coincided with induction of circulating MMP-9 as measured by zymography. Moreover, neutralizing antibodies against MMP-9 prevented IL-8-induced HSC/HPC mobilization (21). Furthermore, Levesque et al., have shown that neutrophil elastase and cathepsin G are involved in G-CSF- and cyclophosphamide-induced HPC mobilization. They found an increased concentration of VCAM-1 cleavage products in the plasma of G-CSF mobilized patients. Concomitantly VCAM-1 expression was decreased and neutrophil elastase and cathepsin G concentrations in the BM were increased (12, 13). Another substrate for neutrophil elastase and cathepsin G was independently identified by two groups who found that SDF-1 (CXCL12) was cleaved leading to a reduced stem cell retention in the BM (10, 11). In addition, neutrophil elastase, cathepsin G, proteinase 3 and matrix metalloproteinase-9 are also capable of cleaving c-kit that is expressed on primitive hematopoietic cells (9). These results suggest that G-CSF-induced mobilization is mediated by interruption of the VCAM-1/VLA-4 pathway through cleavage by proteases that are present in the granules of neutrophils.
In the current study, we show that not only serine proteases (including elastase and cathepsin G), but also serine protease inhibitors play an important role in the induction of HSC/HPC mobilization. Our finding that serpina1 and possibly other serine protease inhibitors play an important role in HSC/HPC mobilization was strengthened by a finding of Winkler et al, who showed a 70- to 1,000-fold reduction in serpina1 and serpina3 mRNA using quantitative RT-PCR and immunoblotting during G-CSF-induced HSC/HPC mobilization in a murine model (33). In addition, a genetic linkage analysis of mobilization in AKXL recombinant inbred strains of mice revealed that a region on chromosome 12 (near 50 cM) close to where the serpina1 gene is located, might be involved in stem cell mobilization (34). Recently, it has been shown that Serpina1 and Serpina3 are involved in hematopoietic progenitor cell mobilization (35). These results explain why the induction of proteases, an event that occurs within hours after G-CSF administration, may initiate HSC/HPC mobilization only when the concentration of the natural protease inhibitors, i.e. serpina1 is concomitantly decreased. The ratio between proteases and protease inhibitors will then increase dramatically in favor of the proteases and results in cleavage of adhesive interactions between stem cells and stromal elements in the BM micro-environment.

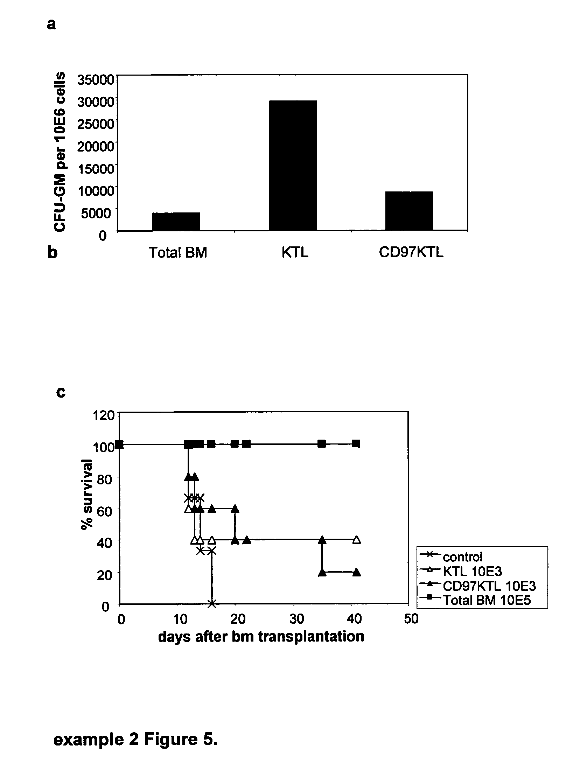

Based on these notions, we propose the following sequence of events during IL-8-induced HSC/HPC mobilization. Upon IL-8 injection, an instant neutropenia occurs due to entrapment of circulating neutrophils in the lungs and probably other organs such as the BM. Underlying this entrapment is the IL-8-induced shedding of L-selectin, and the upregulation of the b2-integrins, LFA-1 and Mac-1 (CD11b), with subsequent firm adhesion to the endothelium. Once attached to the endothelium, neutrophils degranulate in response to activation by IL-8 and release MMP-9 and serine proteases, i.e. neutrophil elastase and cathepsin G. Besides serine proteases, also serine protease inhibitors reside in the BM. One of these, serpina1, is known to be a substrate for MMP-9 (36). Cleavage of serpina1 by MMP-9 results in inactivation of this serine protease inhibitor. In addition, the release of large quantities of chlorinated oxidants that bind to serpina1 also reduces its inhibitory capacity (37). We hypothesize that during IL-8-induced HSC/HPC mobilization MMP-9 is released by neutrophils, an event that leads to the inactivation of serpina1 (figure 5). Serpina1 would otherwise bind to neutrophil elastase to inhibit this serine protease irreversibly. However, its inactivation by MMP-9 increases the ratio between elastase and serpina1 and results in a net increase in neutrophil elastase activity in the BM micro-environment. This leads to disruption of adhesive interactions, and probably also to the degradation of the basement membrane and of the matrix molecules to which hematopoietic stem cells are attached in the micro-environment. In steady state, when the serine protease/serine protease inhibitor ratio favours the protease inhibitors, no HSC/HPC mobilization will occur.
We therefore propose that the delicate balance between serine proteases and their inhibitors is decisive in the initiation of cytokine-induced HSC/HPC mobilization.

### Materials and methods

### Animals

Eight to 12-week-old male Balb/c mice, purchased from Charles River (Maastricht, The Netherlands), were used in all mobilization studies. The animals were maintained in the animal facilities of the Leiden University Medical Center under conventional conditions. All experimental protocols were approved by the institutional ethical committee on animal experiments.

### Stem cell mobilization

Mobilization of HSC was induced by a single intraperitoneal (i.p.) injection of 30 µg of IL-8 (Novartis Research Institute, Vienna, Austria) diluted in endotoxin-free phosphate-buffered saline (PBS) with 0.1% bovine serum. After 20 minutes mice were sacrificed by CO2 asphyxiation and peripheral blood and BM cells were obtained.
G-CSF-induced mobilization was studied by i.p. administration of 10 µg G-CSF per mouse per day (Filgrastim, Neupogen, a kind gift of Amgen, Breda, The Netherlands) for three consecutive days starting at 24 hours after TBI. In inhibition experiments, 300 µg of human serpina1(α1-antitrypsin; Serva Electrophoresis, Heidelberg, Germany) was administered at 2 hours and at 5 minutes prior to IL-8 administration. Serpina1 was heat-inactivated by boiling for 6 minutes at 96°C. Heat-inactivated serpina1 did not inhibit elastase activity in the elastase substrate conversion assay. Colony Forming Units-Granulocyte Macrophage (CFU-GM) were cultured as described previously (24).

### Irradiation protocol

Mice were irradiated in perspex chambers with a linear accelerator (Philips SL 75-5/6 mV, Philips Medical Systems, Best, The Netherlands) at a dose rate of 98 cGy/min. Total doses of 0 (sham irradiated) or 0.5 Gy were administered.

### Elastase activity assay

BMEE were obtained by flushing femurs with 250 µl cold PBS. The cell suspension was centrifuged at 2,300 g for 5 minutes and the supernatant was stored at -20 °C. Elastase activity was determined using the chromogenic substrate N-Succinyl-L-Ala-Ala-Ala-P-nitroanilide (Sigma, Zwijndrecht, The Netherlands). To test for the presence of protease inhibitors, a standard amount of purified porcine pancreas elastase (Sigma) was added to the BMEE immediately before addition of the elastase substrate. Elastase activity was quantified using an elastase standard, during each individual test.

### Preparation of anti-serpina1 antibodies

Anti-serpina1 antibody was prepared by purifying serpina1 from pooled normal mouse serum (Balb/C, C57BL/10 and B10; Janssen Biochimica, Belgium). Serum was adjusted to pH 5.5 by adding one volume 0.5 M sodium acetate pH 5.5 and salted out by adding ammonium sulfate to 1.95 M. After 2 hours at room temperature the supernatant was recovered by centrifugation (4000 g, 20 minutes), and the precipitate was washed with 1.95 M ammonium sulphate. The combined supernatants were dialysed extensively against 1.75 M ammonium sulphate at 4°C and applied to a thiophilic adsorbent (Affi-T agarose; Kem En Tec, Denmark) equilibrated in 1.75 M ammonium sulfate (25, 26). Using a flow of approximately 0.5 ml/min, the adsorbent was washed to its baseline with the same buffer and eluted with 1.5 M ammonium sulfate followed by 0.1 M NaCl. After analysing fractions by crossed immunoelectrophoresis with anti-human serpina1 antibodies (anti-α-1 antitrypsin; Dako A012), which cross-reacted slightly with mouse serpina1, and SDS-PAGE, serpina1 was found in high purity in the 1.5 M ammonium sulphate fraction as a single sharp band migrating at 53 kD in reducing SDS-PAGE. Contrapsin and albumin constituted the main protein impurities. Albumin traces were removed by absorbing the preparation with Blue Sepharose (BioRad, Richmond, CA, USA).

This preparation was used for immunization of rabbits. Bleedings containing the desired antibody reactivity were pooled and antibodies semipurified by a conventional salting-out method (27). The resulting antibody was tested on a blot of mouse serum and reacted with a band having Mr 53 kD. Since the serpina1 that is used for the immunizations is obtained from pooled normal mouse serum, the antibody likely recognizes all 5 mouse serpina1 proteins.

### Western blotting

Ten µg of protein was loaded on a 14% acryl/bisacrylamide gel and subsequently transferred onto an Immobilon-P transfer membrane filter (Millipore, Bedford, MA, USA). Membranes were incubated with 1% blocking reagent (Roche Diagnostics, Mannheim, Germany) and were next incubated with polyclonal rabbit anti-mouse serpina1 antibodies, followed by horseradish peroxidase-polyclonal anti-rabbit IgG antibodies (Promega, Madison, WI, USA) and revealed with the enhanced chemoluminescence method. Densiometry was performed using Eagle-sight software and the irradiated extract vs. sham irradiated extract ratio was calculated for each sample.

### Real time PCR

For real time PCR experiments, BM cells were collected and RNA from 5 x 106 cells was isolated using a Qiagen RNAeasy Mini kit (Westburg, Leusden, The Netherlands) and cDNA was. Subsequently, cDNA was synthesized. The following primer pairs were used: for serpina1 (Forward (F): CAACACCTCCTCCAAACC, Reverse (R): CAGAAACTTCTCCACCAGC), for housekeeping genes HPRT (F: GACTTGCTCGAGATGTCA, R: TGTAATCCAGCAGGTCAG), Beta-Actin (F: AGACCTCTATGCCAACACAG, R: TAGGAGCCAGAGCAGTAATC), and GAPDH (F: ATGGCCTTCCGTGTTCCTAC, R: CCTGCTTCACCACCTTCTT). Data was analyzed using the manufacturer's software and quantified using the comparative CT-method (2-DDCt) that calculates changes in expression relative to the expression of housekeeping genes. All reactions were checked for aspecific products by analyzing the melting curve of the reaction.

### Statistical analysis

Differences were evaluated using the Student's t-test. P values of <0.05 were considered statistically significant.

### Example 2

### Materials and methods

### Animals

Eight to 12-week-old male Balb/c mice, purchased from Charles River (Maastricht, The Netherlands), were used in all mobilization studies. The animals were fed commercial rodent chow and acidified water ad libitum and were maintained in the animal facilities of the Leiden University Medical Center under conventional conditions. All experimental protocols were approved by the institutional ethical committee on animal experiments. Antibodies for cell sorting and analysis
CD97 expression was assessed by flow cytomeytry using a biotinylated hamster anti-murine CD97 mAb (clone 1B2)(1) directed against the first EGF-like domain of mCD97 and was stained with streptavidine-APC or streptavidin-PE. To analyze the phenotype of CD97 subpopulations, cells were stained with FITC-labeled anti-CD8 (clone 53-6.7), anti-TER119 (clone Ter-119), anti-CD4 (clone GK1.5), anti-B220 (clone RA3-6B2), anti-CD11b (clone M1/70.15.11.5), anti-GR-1 (clone RB6-8C5) and with PE-labeled anti-CD117 (c-Kit, clone...; all obtained from Pharmingen, San Diego, CA). Biotinylated anti-CD90.2 (Thy1.2, clone 53-2.1) was stained with commercially available antibodies.

### Preparation of cell suspensions

Using sterile procedures, BMC were obtained by flushing femoral, tibial and humeral bones from donor mice with RPMI medium (Life Technologies, Paisley, Scotland) supplemented with 2 % heat inactivated fetal calf serum, 10% heparine, penicillin and streptomycin. After incubation with DNA-se (1.33 mg/ml), erythrocytes were lysed. Subsequently, the cells were directly used in experiments (total bone marrow cells) or sorted on a FACS Vantage (Beckton Dickinson) before analysis. All populations used in experiments were of >95% purity.

### Progenitor cell assays

Colony Forming Units-Granulocyte Macrophage (CFU-GM) were cultured as described previously(2). Briefly, peripheral blood mononuclear cells were cultured in 3.5-cm dishes containing 5 x 10⁵ cells per ml in semisolid medium in the presence of recombinant murine GM-CSF (1.25 ng/ml, Pharmingen, San Diego, CA, USA). Bone marrow cells were cultured in concentrations of 5 x 104 cells per ml. After 6 days of culture in a fully humidified atmosphere of 37 °C containing 5% CO2, the number of colonies (defined as an aggregate of ≥20 cells) were scored using an inverted light microscope.

### Cobblestone area forming cell (CAFC) assay

Confluent stromal layers of FBMD-1 cells in flat-bottom 96-well plates (Falcon, Etten-Leur, The Netherlands) were overlaid with various dilutions of freshly sorted CD97HI, CD97INT or CD97NEG cells or unseparated BMC to allow limiting dilution analysis of the precursor cells forming hematopoietic clones under the stromal layers. To assay a particular cell suspension, we used 12 dilution steps differing with a factor of 2.5, with 15 wells per dilution. The cells were cultured at 33 °C, 10 % CO2 and were fed weekly by changing half of the medium. Between 7 and 42 days after overlay, all wells were inspected at weekly intervals and scored positive if at least one phase-dark hemopoietic clone (cobblestone area, at least 5 cells) was observed. The CAFC frequencies were calculated using Poisson statistics.

### BMC transplantation

Recipient mice were irradiated in perspex chambers with a linear accelerator (Philips SL 75-5/6 mV, Philips Medical Systems, Best, The Netherlands) at a dose rate of 98 cGy/min. Total doses of 9.5 Gy (lethal irradiation) were administered. Four to 8 hours following total body irradiation, bone marrow cells were injected via caudal vein injection in 0.2 ml of saline, containing 0.2% bovine serum albumin.

### Results

### The majority of hematopoietic progenitor cells are present in the CD97NEG bone marrow cell fraction

To evaluate the expression of CD97 on murine bone marrow cells, primary BALB/c bone marrow cells were stained with anti-CD97 monoclonal antibodies. FACS analysis showed three major populations i.e. CD97HI, CD97INT and CD97NEG cells (average of 71.5%, 24.4% and 4.4% of total BM cells respectively; fig 1a). Based on CD97 expression, bone marrow cells were sorted into CD97HI, CD97INT and CD97NEG populations (figure 1b). Subsequently, CFU-GM colony-forming capacity was analyzed in the different CD97 subsets (n=6). A 2.7-fold enrichment for colony-forming (CFU-GM) capacity was found in the CD97NEG population compared to total bone marrow cells (8660.6 ± 7692.0 CFU-GM vs. 3203.7 ± 1527.2 CFU-GM per 106 bone marrow cells respectively). CD97HI contained few CFU-GM-forming cells (178.4 ± 169.6 CFU-GM per 106 bone marrow cells), whereas CD97INT bone marrow cells exhibited a colony-forming capacity that was comparable to total bone marrow (3047.0 ± 2902.0 CFU-GM per 106 BM cells; fig 1c). These results indicate that the majority of hematopoietic progenitor cells are present in the CD97NEG bone marrow cell fraction

### Repopulating hematopoietic stem cells are resided in the CD97INT bone marrow cell subset

To enummerate the frequency primitive progenitor cells with long-term repopulating ability in the CD97HI, CD97INT and CD97NEG population, sorted BALB/c bone marrow cells were tested in a cobblestone area forming cell assay. The majority of CAFC-day 7, containing solely cells with progenitor activity, are found in the CD97NEG fraction compared to CD97HI, CD97INT and total bone marrow cells (852 ± 60.7 CAFC-day 7 per 10⁵ cells vs. 0.23 ± 0.0, 201.2± 260.0 and 107.6 ± 17.9 CAFC per 10⁵ cells respectively; figure 2a). However, the frequency of CAFC decreased during the weeks of culture and no CAFC-dag 28 were present in the CD97NEG fraction. The CD97HI BM fraction contained very few CAFC throughout the whole culturing period and no CAFC were found after 28 days of culturing. In contrast, the CD97INT bone marrow cells showed a high frequency of CAFC-day 7, compared to total bone marrow cells, and the frequency remained high throughout the entire culturing period (201.2 ± 260.0, 186.7 ± 23.9, 118.2 ± 8.9, 36.5 ±12.2 and 3.1 ± 0.1 CAFC per 10⁵ cells for CAFC-day 7, 14, 21, 28 and 35 respectively (figure 2a).
Subsequently, to investigate the repopulating ability of the different CD97 sorted bone marrow cell fractions, 1 x 10⁵ CD97 sorted bone marrow cells were transplanted into lethally irradiated (9.5 Gy) syngeneic recipient mice. None of the mice that were transplanted with CD97HI or CD97NEG bone marrow cells (n=10 per group) and none of control the mice that received total body irradiation (TBI) alone (n=6) were alive at 8 weeks following transplantation. However mice that received 1 x 10⁵ CD97INT bone marrow cells (n=10) survived >120 days following transplantation (figure 2b). To confirm that CD97INT bone marrow cells contained life-long repopulating ability, lethally irradiated (9.5 Gy) secondary recipient mice were transplanted with 1 x 10⁵ total bone marrow cells obtained from mice that had previously received 1 x 10⁵ CD97INT bone marrow cells or from recipients of 1 x 10⁵ total bone marrow cells. At 100 days following transplantation, 4/5 mice that were secondary recipients of CD97INT bone marrow cells and 4/5 secondary recipients of 1 x 10⁵ total bone marrow cells were alive (figure 2c). There results indicate that the CD97INT bone marrow cell fraction contains hematopoietic stem cells with long-term repopulating ability.

### CD97INT cells are c-KitHIThy-1LOLinNEGWGA+

In mice, hematopoietic stem cells are characterized as c-KitHIThy-1LOLinNEG (ref). To evaluate the presence of c-KitHIThy-1LOLinNEG cells in CD97HI, CD97INT and CD97NEG bone marrow cell fractions, total unseparated bone marrow cells were stained for c-Kit, Thy-1 and Lineage (Lin) markers. The majority of CD97HI expressing cells are positive for Lin markers, negative for Thy-1 and do not express c-Kit. In contrast, a supopulation of both CD97INT and CD97NEG bone marrow cells are c-KitHIThy-1lo and are negative for Lin markers (figure 3a). Another phenotypical marker for hematopoietic stem cells is wheat germ agglutinin (WGA). This molecule binds to sialic residues of glycoproteins and expressed hematopoietic stem cells (ref). FACS analysis revealed that CD97HI cells are negative for WGA-staining, while both CD97INT and CD97NEG bone marrow cells contain a c-KITHIWGA+ population (figure 3a). In another experiment, unsorted bone marrow cells were selected for c-KitHI, Thy-1LO and LinNEG cells. Subsequently, the expression of CD97 was analyzed within this population of cells. The majority of c-KitHIThy-1LOLinNEG bone marrow cells (51.6%) are present in the CD97INT fraction, whereas only 15.3% of the CD97NEG and 33.7% of the CD97HI bone marrow cells are resided in the c-KitHIThy-1LOLinNEG population (figure 3b).

### CD97INTc-KitHIThy-1LO cells are hematopoietic stem cells

The isolation of murine hematopoietic stem cells is a multistep process, consisting of both magnetic and fluorescent cell sorting steps (ref). To investigate whether the isolation of hematopoietic stem cells can be simplified, by selecting for CD97 expression, CD97INTc-KitHIThy-1LO and CD97INTc-KitHIThy-1LOLinNEG bone marrow cells were analyzed for progenitor cell content and repopulating ability in-vitro and in-vivo.

### Data C57BL/6

FACS analysis has revealed that the donor BM cells were responsible for the reconstitution of the host.

### Long-term repopulating ability hematopoietic stem cells can be isolated according to CD97 and c-KIT expresssion

To investigate whether the isolation of long-term repopulating ability hematopoietic stem cells can be simplified, we isolated CD97INTc-KitHI bone marrow cells and investigated the HSC and HPC content of these populations as well as the repopulating ability in-vivo.

Reference List
1. Bensinger, W. I., Martin, P. J., Storer, B., Clift, R., Forman, S. J., Negrin, R., Kashyap, A., Flowers, M. E., Lilleby, K., Chauncey, T. R. et al. (2001) N. Engl. J. Med. 344, 175-181.
2. Champlin, R. E., Schmitz, N., Horowitz, M. M., Chapuis, B., Chopra, R., Cornelissen, J. J., Gale, R. P., Goldman, J. M., Loberiza, F. R., Jr., Hertenstein, B. et al. (2000) Blood 95, 3702-3709.
3. Powles, R., Mehta, J., Kulkarni, S., Treleaven, J., Millar, B., Marsden, J., Shepherd, V., Rowland, A., Sirohi, B., Tait, D. et al. (2000) Lancet 355, 1231-1237.
4. Lieschke, G. J. & Burgess, A. W. (1992) N. Engl. J. Med. 327, 99-106.
5. Bensinger, W. I., Price, T. H., Dale, D. C., Appelbaum, F. R., Clift, R., Lilleby, K., Williams, B., Storb, R., Thomas, E. D. & Buckner, C. D. (1993) Blood 81, 1883-1888.
6. Laterveer, L., Lindley, I. J., Hamilton, M. S., Willemze, R. & Fibbe, W. E. (1995) Blood 85, 2269-2275.
7. Teixido, J., Hemler, M. E., Greenberger, J. S. & Anklesaria, P. (1992) J. Clin. Invest 90, 358-367.
8. Oostendorp, R. A., Reisbach, G., Spitzer, E., Thalmeier, K., Dienemann, H., Mergenthaler, H. G. & Dormer, P. (1995) Br. J. Haematol. 91, 275-284.
9. Levesque, J. P., Hendy, J., Winkler, I. G., Takamatsu, Y. & Simmons, P. J. (2003) Exp. Hematol. 31, 109-117.
10. Petit, I., Szyper-Kravitz, M., Nagler, A., Lahav, M., Peled, A., Habler, L., Ponomaryov, T., Taichman, R. S., Arenzana-Seisdedos, F., Fujii, N. et al. (2002) Nat. Immunol. 3, 687-694.
11. Levesque, J. P., Hendy, J., Takamatsu, Y., Simmons, P. J. & Bendall, L. J. (2003) J. Clin. Invest 111, 187-196.
12. Levesque, J. P., Takamatsu, Y., Nilsson, S. K., Haylock, D. N. & Simmons, P. J. (2001) Blood 98, 1289-1297.
13. Levesque, J. P., Hendy, J., Takamatsu, Y., Williams, B., Winkler, I. G. & Simmons, P. J. (2002) Exp. Hematol. 30, 440-449.
14. Papayannopoulou, T. & Nakamoto, B. (1993) Proc. Natl. Acad. Sci. U. S. A 90, 9374-9378.
15. Craddock, C. F., Nakamoto, B., Andrews, R. G., Priestley, G. V. & Papayannopoulou, T. (1997) Blood 90, 4779-4788.
16. Laterveer, L., Lindley, I. J., Heemskerk, D. P., Camps, J. A., Pauwels, E. K., Willemze, R. & Fibbe, W. E. (1996) Blood 87, 781-788.
17. Pruijt, J. F., Verzaal, P., van Os, R., de Kruijf, E. J., van Schie, M. L., Mantovani, A., Vecchi, A., Lindley, I. J., Willemze, R., Starckx, S. et al. (2002) Proc. Natl. Acad. Sci. U. S. A 99, 6228-6233.
18. Pruijt, J. F., van Kooyk, Y., Figdor, C. G., Lindley, I. J., Willemze, R. & Fibbe, W. E. (1998) Blood 91, 4099-410⁵.
19. Pruijt, J. F., van Kooyk, Y., Figdor, C. G., Willemze, R. & Fibbe, W. E. (1999) Blood 93, 107-112.
20. Morrison, S. J., Wandycz, A. M., Hemmati, H. D., Wright, D. E. & Weissman, I. L. (1997) Development 124, 1929-1939.
21. Pruijt, J. F., Fibbe, W. E., Laterveer, L., Pieters, R. A., Lindley, I. J., Paemen, L., Masure, S., Willemze, R. & Opdenakker, G. (1999) Proc. Natl. Acad. Sci. U. S. A 96, 10863-10868.
22. Lindley, I., Aschauer, H., Seifert, J. M., Lam, C., Brunowsky, W., Kownatzki, E., Thelen, M., Peveri, P., Dewald, B., von, T., V et al. (1988) Proc. Natl. Acad. Sci. U. S. A 85, 9199-9203.
23. Zwierzina, H., Holzinger, I., Gaggl, S., Wolf, H., Schollenberger, S., Lam, C., Bammer, T., Geissler, D. & Lindley, I. (1993) Scand. J. Immunol. 37, 322-328.
24. Fibbe, W. E., Hamilton, M. S., Laterveer, L. L., Kibbelaar, R. E., Falkenburg, J. H., Visser, J. W. & Willemze, R. (1992) J. Immunol. 148, 417-421.
25. Porath, J., Maisano, F. & Belew, M. (1985) FEBS Lett. 185, 306-310.
26. Lihme, A. & Heegaard, P. M. (1991) Anal. Biochem. 192, 64-69.
27. Harboe, N. & Ingild, A. (1973) Scand. J. Immunol. Suppl 1, 161-164.
28. Korzus, E., Dubin, A., Potempa, J. & Travis, J. (1991) Biomed. Biochim. Acta 50, 687-690.
29. Wright, H. T. (1996) Bioessays 18, 453-464.
30. Sottrup-Jensen, L. (1989) J. Biol. Chem. 264, 11539-11542.
31. Silverman, G. A., Bird, P. I., Carrell, R. W., Church, F. C., Coughlin, P. B., Gettins, P. G., Irving, J. A., Lomas, D. A., Luke, C. J., Moyer, R. W. et al. (2001) J. Biol. Chem. 276, 33293-33296.
32. Carrell, R. W. & Lomas, D. A. (2002) N. Engl. J. Med. 346, 45-53.
33. Winkler, I. G., Hendy, J., Horvath, A., Coughlin, P. & Levesque, J. P. Transcriptional repression of serpins (serine protease inhibitors) enhances active protease levels in the bone marrow during haematopoietic stem cell mobilization. Exp.Hematol. 31, 152 abstract no 275-152. 2003.
34. de Haan, G., Ausema, A., Wilkens, M., Molineux, G. & Dontje, B. (2000) Br. J. Haematol. 110, 638-646.
35. Winkler, I. G., Hendy, J., Coughlin, P., Horvath, A. & Levesque, J. P. (2005) J. Exp. Med. 201, 1077-1088.
36. Liu, Z., Zhou, X., Shapiro, S. D., Shipley, J. M., Twining, S. S., Diaz, L. A., Senior, R. M. & Werb, Z. (2000) Cell 102, 647-655.
37. Weiss, S. J. & Regiani, S. (1984) J. Clin. Invest 73, 1297-1303.

### Brief description of the drawings

Example 1 Figure 1. IL-8-induced and G-CSF-induced HSC/HPC mobilization is inhibited after low dose total body irradiation. Mice received 0.5 Gy TBI or were sham irradiated and subsequently mobilized with IL-8 (30 mg/mouse; n=12), G-CSF (n=10) or with PBS (n=11) as a control. Colony-forming capacity of peripheral blood (a) and BM (b) are depicted. Results are shown as mean ± SD. *p<0.0001 vs 0 Gy/IL-8, **p<0.0001 vs 0 Gy/G-CSF.

Example 1 Figure 2. (a) Inhibition of elastase activity by BMEE obtained from irradiated mice. Low dose (0.5 Gy; n=9) irradiated mice were sacrificed 24 hrs after TBI and BMEE were obtained. Elastase activity was measured in BMEE both in the absence as well as in the presence of additional elastase. *p<0.05 as compared to BMEE obtained from sham irradiated controls. (b) Elastase activity can be restored by anti-serpina1 antibodies. BMEE obtained from low dose (0.5 Gy) irradiated mice (n=2) were incubated with 3.125 mg/ml elastase in the absence and presence of anti-serpina1 antibodies (Ab, solid and hatched bars respectively). Subsequently, elastase activity was assessed by a chromogenic substrate assay. Results are related to the elastase activity in BMEE obtained from sham irradiated controls.

Example 1 Figure 3. Induction of serpina1 mRNA and protein following low dose TBI. (a)Real time PCR for serpina1 mRNA analysis of total BM cells at 24 hours following 0.5 Gy TBI (white bar; n=2). Serpina1 mRNA is increased 7-fold at 24 hours after 0.5 Gy TBI relative to HPRT expression (left Y-axis). BM supernatants obtained from 0.5 Gy irradiated (black bar; n=7) mice were analyzed for the presence of serpina1 protein by Western blotting. The relative concentration of serpina1protein was assessed by optical density measurement of each band and related to sham irradiated mice of the same blot (right Y-axis). (b) Ten mg of total protein from BM supernatants obtained from sham irradiated and 0.5 Gy irradiated mice were analyzed for the presence of serpina1 protein by Western blotting. The relative concentration of serpina1 was assessed by optical density measurement of each band.

Example 1 Figure 4. Serpina1 inhibits IL-8-induced HSC/HPC mobilization. At two hours and at 5 minutes prior to IL-8-induced HSC/HPC mobilization, human serpina1 (300 mg) was administered to the mice by i.p. injection. Control mice received PBS or heat-inactivated serpina1 (n=4). Subsequently, the mice were mobilized with IL-8 (30 mg/mouse; n=14) or received PBS as a control (n=9) and the number of colony forming units (CFU-GM) was evaluated in (a) peripheral blood and (b) BM. Results from 4 independent experiments are shown as mean ± SD. * p<0.05 compared to IL-8-treated control mice.

Example 1 Figure 5. Schematic representation of the role of neutrophils, proteases and protease inhibitors during IL-8-induced HSC/HPC mobilization. Upon IL-8 injection, neutrophils upregulate cell adhesion molecules, including LFA-1 and Mac-1, with subsequent rolling and firm adhesion to the endothelium. (I) Once attached to the endothelium, neutrophils degranulate in response to activation by IL-8 and release MMP-9 and serine proteases, including neutrophil elastase. Serine protease inhibitors, including serpina1, are present in high concentrations in plasma. (II) When the protease/protease inhibitor ratio favors the proteases (excess protease), the cytoadhesive interactions between HSC/HPC and stromal elements will be degraded. (III) Subsequently, HSC/HPC mobilize to the peripheral blood. (IV, V) Induction of protease inhibitors in the BM results in excess inhibitor (VI) and no HSC/HPC mobilization will occur. Upper panel. Positive and negative regulation of protease activity. Cleavage of serpina1 (alpha-1 antitrypsin, α-1 AT) by MMP-9 results in inactivation of this serine protease inhibitor. Serpina1would normally bind to neutrophil elastase to inhibit this serine protease. However, its inactivation results in a netto increase in neutrophil elastase activity in the BM micro-environment. Elastase, in turn, inactivates MMP-9, resulting in less inhibition of serpina1. Besides inactivation of serpina1, MMP-9 cleaves IL-8 into a molecule with a higher bioactivity. The presence of more bioactive IL-8 leads to higher levels of MMP-9.

Example 2 Figure 1. Murine bone marrow can be divided into three major populations according to CD97 expression. (a) Murine bone marrow cells were stained with anti-CD97 mAb and CD97 expression was evaluated by flowcytometric analysis. (b) Based on CD97 expression bone marrow was sorted into CD97^{HI}, CD97^{INT} and CD97^{NEG} populations. The percentages indicate the cell fractions defined by the gates shown. (c) Colony-forming capacity of each CD97-sorted bone marrow cell population was analyzed. Results of 6 independent experiments are shown as mean +/- SD.

Example 2 Figure 2. Hematopoietic stem cell activity is resided in the CD97^{INT} population. (a) Bone marrow cells were sorted according to CD97 expression into CD97^{HI}, CD97^{INT} and CD97^{NEG} populations. Each sorted population was analyzed for CAFC-forming capacity during 5 weeks. Results of 3 independent experiments are shown as mean +/- SD. (b) Radioprotection by 1 x10⁵ CD97-sorted bone marrow cells. Lethally irradiated recipients (9.5 Gy; n=10 per group) were transplanted with 1 x 10⁵ total bone marrow cells or 1 x 10⁵ purified CD97^{HI}, CD97^{INT} and CD97^{NEG} bone marrow cells. (c) Radioprotection by 1 x10⁵ sorted bone marrow cells in 2^{nd} recipients. Lethally irradiated recipients of 1 x 10⁵ CD97^{INT} transplanted bone marrow cells were sacrificed on day 126 after bone marrow transplantation and bone marrow cells were harvested. Subsequently, 1 x 10⁵ total bone marrow cells were administered to lethally irradiated (9.5 Gy) 2^{nd} recipients (n=5 per group).

Example 2 Figure 3. Phenotypical analysis of unsorted BALB/c bone marrow cells. (a) CD97^{INT} and CD97^{NEG} cells exhibit hematopoietic stem cell phenotype. Total bone marrow cells were divided into CD97^{HI}, CD97^{INT} and CD97^{NEG} subpopulations based on CD97 expression. Each subpopulation was analyzed for Thy-1, lineage markers (Lin) and Wheat Germ Agglutinin (WGA) in comparison to c-Kit. (b) The majority of c-KIT^{HI}Thy-1^{LO}Lin^{NEG} cells is resided in the CD97^{INT} population. Total bone marrow cells were stained for c-Kit, Thy-1, Lin-markers and CD97. The c-KIT^{HI}Thy-1^{LO}Lin^{NEG} cells were gated and analyzed for CD97 expression.

Example 2 Figure 4. Repopulating ability in-vitro and in-vivo of c-Kit^{HI}Thy-1^{LO} and CD97^{INT}c-Kit^{HI}Thy-1^{LO} bone marrow cells. (a) CFU-GM analysis of sorted bone marrow cells. (b) CAFC assay of c-Kit^{HI}Thy-1^{LO}and CD97^{INT}c-Kit^{HI}Thy-1^{LO} bone marrow cells in comparison with total bone marrow cells. KT are c-Kit^{HI}Thy-1^{LO} cells. (c) Recipient survival at 60 days following lethal irradiation (9.5 Gy) and subsequent bone marrow cell transplantation (n=3-10 per group). CD97KT are CD97^{INT}c-Kit^{HI}Thy-1^{LO} cells.

Example 2 Figure 5. Repopulating ability in-vitro and in-vivo of c-Kit^{HI}Thy-1^{LO}Lin^{NE}G and CD97^{INT}c-Kit^{HI}Thy-1^{LO}Lin^{NEG} bone marrow cells. (a) CFU-GM analysis of sorted bone marrow cells. (b) CAFC assay of c- Kit^{HI}Thy-1^{LO}Lin^{NEG} and CD97^{INT}c-Kit^{HI}Thy-1^{LO}Lin^{NEG} bone marrow cells in comparison with total bone marrow cells. KTL are c-Kit^{HI}Thy-1^{LO}Lin^{NEG} cells. (c) Recipient survival following lethal total body irradiation (9.5 Gy) and subsequent bone marrow cell transplantation (n=3-5 per group). CD97KTL are CD97^{INT}c-Kit^{HI}Thy-1^{LO} cells.

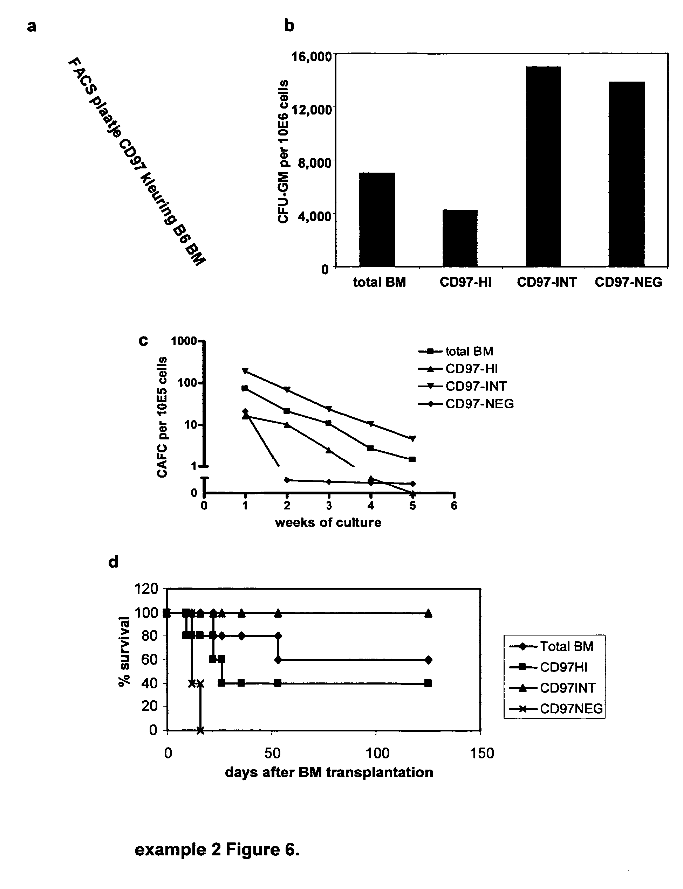

Example 2 Figure 6. CD97^{INT} bone marrow cells have repopulating ability in-vitro and in-vivo in C57BL/6 mice. (a) C57BL/6 bone marrow cells were stained with anti-CD97 mAb and CD97 expression was evaluated by flowcytometric analysis. (b) Based on CD97 expression bone marrow was sorted into CD97^{HI}, CD97^{INT} and CD97^{NEG} populations. Colony-forming (CFU-GM) capacity of each CD97-sorted bone marrow cell population was analyzed. (c) Bone marrow cells were sorted according to CD97 expression into CD97^{HI}, CD97^{INT} and CD97^{NEG} populations. Each sorted population was analyzed for CAFC-forming capacity during 5 weeks. (d) Radioprotection by 1 x 10⁵ CD97-sorted C57BL/6 (Ly5.1) bone marrow cells. Lethally irradiated (9.5 Gy) recipients (C57BL/6, Ly5.2; n=5 per group) were transplanted with 1 x 10⁵ total bone marrow cells or 1 x 10⁵ purified CD97HI, CD97^{INT} and CD97^{NEG} bone marrow cells.

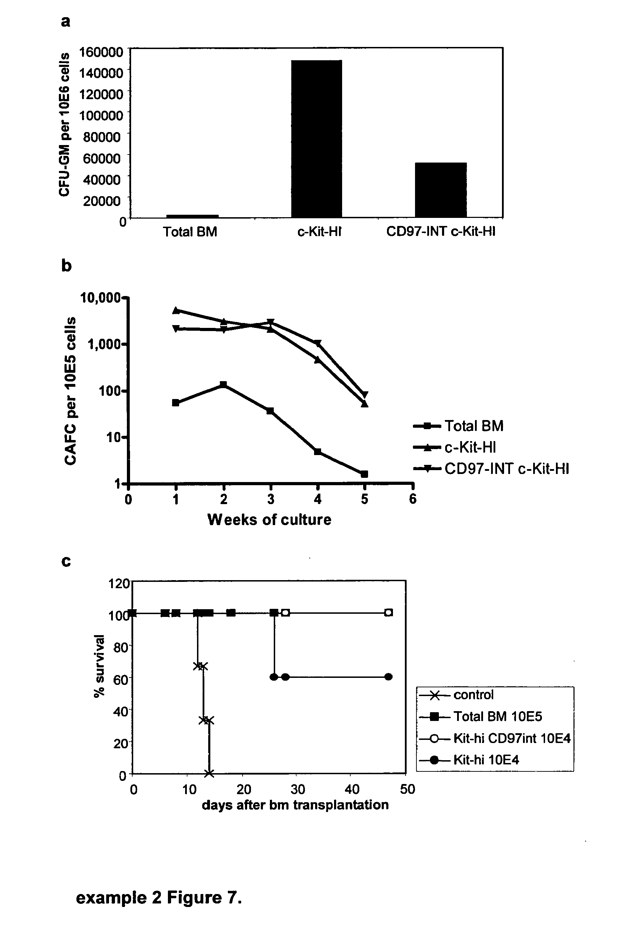

Example 2 Figure 7. Repopulating ability in-vitro and in-vivo of c-Kit^{HI} and CD97^{INT}c-Kit^{HI} bone marrow cells. (a) CFU-GM analysis of sorted bone marrow cells. (b) CAFC assay of c-Kit^{HI} and CD97^{INT}C-Kit^{HI} bone marrow cells in comparison with total bone marrow cells. (c) Recipient survival following lethal total body irradiation (9.5 Gy) and subsequent bone marrow cell transplantation (n=3-5 per group).

## Claims

1. A method for modulating mobility of a stem cell in an individual, said method comprising modulating elastase activity in the vicinity of said stem cell.

2. A method according to claim 1, wherein said mobility is increased by increasing elastase activity in the vicinity of said stem cell.

3. A method according to claim 2, wherein said increase is achieved by providing said individual with an inhibitor of a protease inhibitor.

4. A method according to claim 3, wherein said inhibitor comprises a binding body specific for said protease inhibitor.

5. A method according to claim 3 or claim 4, wherein said protease inhibitor comprises serpina1 or α2-MG.

6. A method according to any one of claims 1-5, wherein said stem cell is a hemopoietic stem cell.

7. Use of a binding body specific for a protease inhibitor, or a functional part, derivative and/or analogue of said antibody for stimulating mobility of a stem cell in an individual comprising said stem cell.

8. A composition comprising a growth factor or cytokine for stimulating mobilization of stem cells in an individual and an inhibitor of serpina1.

9. A composition according to claim 8, wherein said growth factor comprises C-GSF.

10. A method for identifying a cell in a population of cells, said method comprising contacting said population of cells with a binding body specific for CD97 and identifying said cell on the basis of the relative amount of binding body associated with said cell.

11. A method according to claim 10, wherein said population of cells comprises a stem cell.

12. A method according to claim 10 or claim 11, further comprising separating said identified cell from another cell in said population of cells.

13. A method according to any one of claims 10-13, wherein said identifying comprises classifying cells in said population of cells as having a high amount, an intermediate amount or no detectable CD97 specific binding body associated therewith.

14. A method according to claim 13, wherein said cell is identified through its classification as a cell having an intermediate level of CD97 specific binding body associated therewith.

15. A method according to any one of claims 10-15, further comprising contacting said population of cells with a binding body specific for CD34 or homologue thereof, specific for CD38 or homologue thereof and/or specific for c-kit or homologue thereof.

16. A method according to claim 15, further comprising separating cells from said population on the basis of the relative amount of binding body specific for CD34, CD38 and/or c-kit or homologues thereof on said cells.

17. A method according to any one of claims 10-16, for marking a subset of cells in a population of cells.

18. A method according to claim 17, for marking a subset that is enriched for stem cells.

19. A kit for separating stem cells from other cells in a population of cells comprising a binding body specific for CD97 or a homologue thereof.

20. A kit according to claim 19, wherein said binding body is associated with a solid surface.

21. A kit according to claim 19 or claim 20, further comprising a binding body for distinguishing stem cells from other cells in said population of cells.
